# EUROPÄISCHE PATENTSCHRIFT

(11) **EP 0 727 463 B1**
(45) Veröffentlichungstag und Bekanntmachung des Hinweises auf die Patenterteilung: **03.05.2000**
(21) Anmeldenummer: 96101637.5
(22) Anmeldetag: 06.02.1996
(51) Int. Cl.: C09B 23/04, C09B 55/00, C07D 471/04, B41M 5/38

(54) **Triazolopyridinfarbstoffe und deren Zwischenprodukte**
Triazolopyridine dyes and intermediate products therefor
Colorants triazolopyridines et leurs produits intermédiares

(30) Priorität: 15.02.1995 DE 19504943
(43) Veröffentlichungstag der Anmeldung: 21.08.1996
(73) Patentinhaber: BASF AKTIENGESELLSCHAFT, 67056 Ludwigshafen (DE)
(72) Erfinder: Sens, Rüdiger, Dr., D-68165 Mannheim (DE); Reichelt, Helmut, Dr., D-67435 Neustadt (DE); Saling, Peter, Dr., D-67434 Neustadt (DE)

(56) Entgegenhaltungen:
- EP-A- 0 413 226
- EP-A- 0 416 434
- EP-A- 0 591 736
- WO-A-95/21219

## Beschreibung

Die vorliegende Erfindung betrifft neue Triazolopyridinfarbstoffe der Formel I 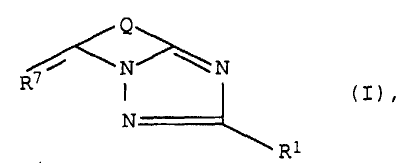 in der
- R¹: C₁-C₂₀-Alkyl, das gegebenenfalls substituiert ist und durch 1 bis 4 Sauerstoffatome in Etherfunktion unterbrochen sein kann, gegebenenfalls substituiertes Phenyl, Hydroxy, gegebenenfalls substituiertes C₁-C₂₀-Alkoxy, Mercapto oder gegebenenfalls substituiertes C₁-C₂₀-Alkylthio,
- Q: einen Rest der Formel 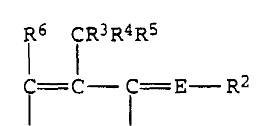 oder 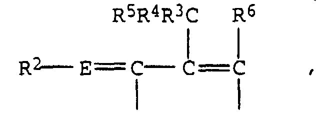 worin
R² für einen 5- oder 6-gliedrigen carbocyclischen oder heterocyclischen Rest, der benzoanelliert sein kann,
R³ für Wasserstoff oder gegebenenfalls substituiertes C₁-C₄-Alkyl,
R⁴ für Wasserstoff, gegebenenfalls substituiertes C₁-C₄-Alkyl oder C₁-C₄-Alkoxy,
R⁵ für C₁-C₈-Alkyl, das gegebenenfalls durch 1 oder 2 Sauerstoffatome in Etherfunktion unterbrochen ist und durch Phenyl oder Hydroxy substituiert sein kann, gegebenenfalls substituiertes Phenyl, gegebenenfalls substituiertes Thienyl oder C₁-C₄-Alkoxy, das gegebenenfalls durch ein Sauerstoffatom in Etherfunktion unterbrochen ist, oder der Rest CR³R⁴R⁵ zusammen C₃-C₇-Cycloalkyl, C₁-C₄-Halogenalkyl, gegebenenfalls substituiertes Phenyl oder gegebenenfalls substitiertes Thienyl,
R⁶ für Cyano, Carbamoyl, Carboxyl, C₁-C₄-Alkoxycarbonyl oder Benzthiazolyl und
E für CH oder Stickstoff stehen, und
- R⁷: Sauerstoff oder einen Rest der Formel 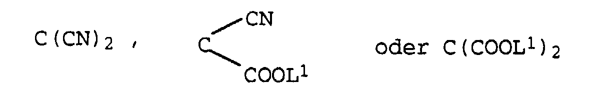
bedeuten, wobei L¹ jeweils für C₁-C₈-Alkyl, das gegebenenfalls durch 1 oder 2 Sauerstoffatome in Etherfunktion unterbrochen ist, steht,
ein Verfahren zum thermischen Transfer dieser Farbstoffe, ihre Verwendung zum Färben oder Bedrucken von synthetischen Materialien sowie Triazolopyridine als Zwischenprodukte für diese Farbstoffe.

Aus der US-A-5 079 365 sowie der WO-A-95/22581 sind Triazolopyridinfarbstoffe bekannt, die in Ringposition 4 des Pyridinrings einen anderen Substituenten tragen.

Aufgabe der vorliegenden Erfindung war es nun, neue Triazolopyridinfarbstoffe mit anderer chemischer Struktur und vorteilhaften Eigenschaften bereitzustellen. Sie sollten in einfacher Weise herzustellen sein.

Demgemäß wurden die eingangs naher bezeichneten Triazolopyridinfarbstoffe der Formel I gefunden.

Die Farbstoffe der Formel I können in mehreren tautomeren Formen auftreten, die alle von den Patentansprüchen umfaßt werden. Beispielsweise können die Verbindungen mit R³ = Wasserstoff und R⁷ = Sauerstoff u.a. in folgenden tautomeren Formen auftreten:

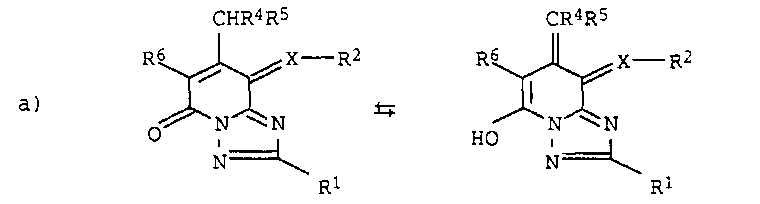

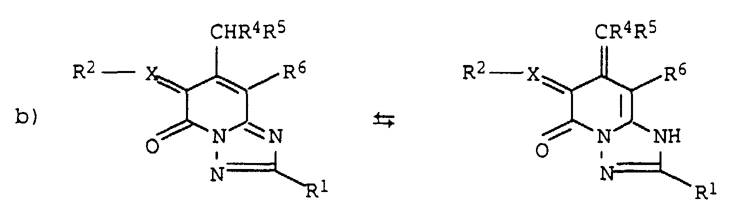

R² stellt einen 5- oder 6-gliedrigen carbocyclischen oder heterocyclischen Rest dar, der gegebenenfalls substituiert ist und benzoanelliert sein kann.

Reste R² können sich z.B. von Komponenten aus der Benzol-, Indol-, Chinolin-, Aminonaphthalin-, Pyrrol-, Aminothiazol-, Benzimidazol-, Benzthiazol-, Aminothiophen- oder Diaminopyridinreihe ableiten.

Wichtige Reste R² sind z.B. solche der Formeln IIa bis IIj 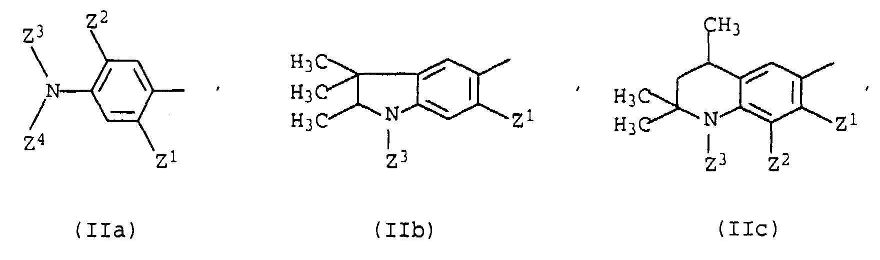 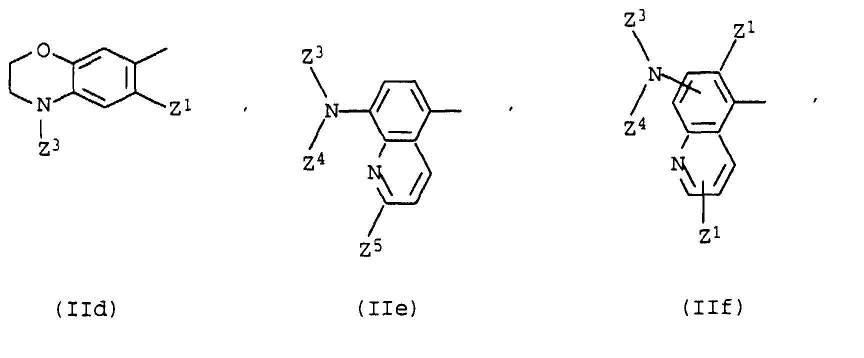 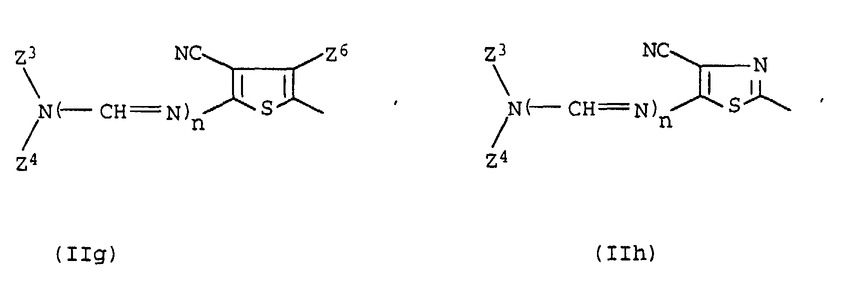 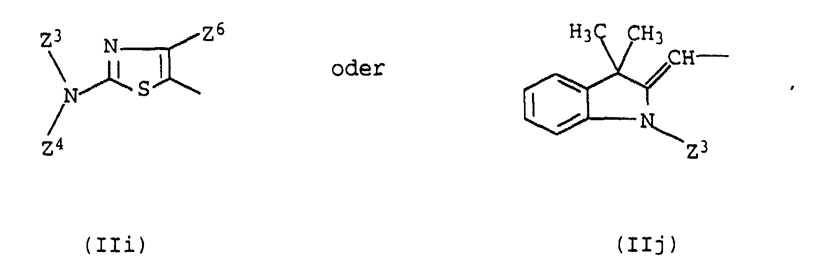 worin
- n: für 0 oder 1,
- Z¹: für Wasserstoff, C₁-C₈-Alkyl, das gegebenenfalls durch 1 oder 2 Sauerstoffatome in Etherfunktion unterbrochen ist, Hydroxy, C₁-C₄-Alkoxy, insbesondere Methoxy oder Ethoxy, Formylamino, C₁-C₄-Alkylsulfonylamino, C₁-C₄-Mono- oder Dialkylaminosulfonylamino oder den Rest -NHCOZ⁷ oder -NHCO₂Z⁷, wobei Z⁷ die Bedeutung von Phenyl, Benzyl, Tolyl oder C₁-C₈-Alkyl, das gegebenenfalls durch 1 oder 2 Sauerstoffatome in Etherfunktion unterbrochen ist, besitzt,
- Z²: für Wasserstoff, C₁-C₄-Alkyl, insbesondere Methyl, oder C₁-C₄-Alkoxy, insbesondere Methoxy oder Ethoxy,
- Z³ und Z⁴: unabhängig voneinander jeweils für Wasserstoff, C₁-C₈-Alkyl, das gegebenenfalls substituiert ist und durch 1 oder 2 Sauerstoffatome in Etherfunktion unterbrochen sein kann, C₃-C₄-Alkenyl, C₅-C₇-Cycloalkyl, gegebenenfalls substituiertes Phenyl oder zusammen mit dem sie verbindenden Stickstoffatom für einen 5- oder 6-gliedrigen gesättigten heterocyclischen Rest, der gegebenenfalls weitere Heteroatome enthält,
- Z⁵: für Wasserstoff oder C₁-C₄-Alkyl, insbesondere Methyl, und
- Z⁶: für Wasserstoff, Halogen, C₁-C₈-Alkyl, gegebenenfalls substituiertes Phenyl, gegebenenfalls substituiertes Benzyl, Cyclohexyl, Thienyl, Hydroxy, C₁-C₄-Alkoxy, C₁-C₄-Alkylthio oder C₁-C₈-Monoalkylamino stehen.

Alle in den hier genannten Formeln auftretenden Alkyl- und Alkenylgruppen können sowohl geradkettig als auch verzweigt sein.

Wenn in den hier genannten Formeln substituierte Alkylreste auftreten, so können als Substituenten z.B. gegebenenfalls substituiertes Phenyl, gegebenenfalls substituiertes Phenoxy, Carboxyl, C₁-C₂₀-Alkoxycarbonyl, dessen Alkylkette gegebenenfalls durch 1 bis 4 Sauerstoffatome in Etherfunktion unterbrochen und durch Phenyl oder Phenoxy substituiert sein kann, Hydroxy, Halogen, Cyano, C₁-C₆-Alkanoyloxy, C₁-C₄-Alkylaminocarbonyloxy oder C₁-C₄-Alkoxycarbonyloxy, wobei im letzten Fall die Alkoxygruppe durch Phenyl oder C₁-C₄-Alkoxy substituiert sein kann, in Betracht kommen. Die Alkylreste weisen dabei in der Regel 1 bis 3 Substituenten auf.

Wenn in den hier genannten Formeln Alkylreste auftreten, die durch Sauerstoffatome in Etherfunktion unterbrochen sind, so sind solche Alkylreste bevorzugt, die durch 1 oder 2 Sauerstoffatome in Etherfunktion unterbrochen sind.

Wenn in den hier genannten Formeln substituierte Phenyl- oder Thienylreste auftreten, so können als Substituenten z.B. C₁-C₄-Alkyl, Trifluormethyl, C₁-C₄-Alkoxy, Halogen, Nitro oder Carboxyl in Betracht kommen. Die Phenyl- oder Thienylreste weisen dabei in der Regel 1 bis 3 Substituenten auf.

Geeignete Reste L¹, R¹, R³, R⁴, R⁵, Z¹, Z², Z³, Z⁴, Z⁵, Z⁶ und Z⁷ sind, ebenso wie die unten genannten Rest L² und L³, z.B. Methyl, Ethyl, Propyl, Isopropyl, Butyl, Isobutyl, sec-Butyl oder tert-Butyl.

Reste L¹, R¹, R⁵, Z¹, Z³, Z⁴, Z⁶ und Z⁷ sind weiterhin z.B. Pentyl, Isopentyl, Neopentyl, tert-Pentyl, Hexyl, 2-Methylpentyl, Heptyl, Octyl, 2-Ethylhexyl oder Isooctyl.

Reste R¹ sind weiterhin z.B. Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl oder Eicosyl. (Die Bezeichnungen Isooctyl, Isononyl, Isodecyl und Isotridecyl sind Trivialbezeichnungen und stammen von den nach der Oxosynthese erhaltenen Alkoholen (vgl. dazu Ullmann's Encyclopedia of Industrial Chemistry, 5th Edition, Vol. A1, Seiten 290 bis 293, sowie Vol. A 10, Seiten 284 und 285)).

Reste L¹, R¹, R⁵, Z¹, Z³, Z⁴ und Z⁷ sind weiterhin z.B. 2-Methoxyethyl, 2-Ethoxyethyl, 2-Propoxyethyl, 2-Butoxyethyl, 2- oder 3-Methoxypropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Butoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, oder 4,8-Dioxadecyl.

Reste R¹ sind weiterhin z.B. 3,6,9-Trioxadecyl, 3,6,9-Trioxaundecyl, 3,6,9,12-Tetraoxatridecyl, 3,6,9,12-Tetraoxatetradecyl, Methylthio, Ethylthio, Propylthio, Isopropylthio, Butylthio, Isobutylthio, sec-Butylthio, Pentylthio, Isopentylthio, Neopentylthio, tert-Pentylthio, Hexylthio, Heptylthio, 1-Ethylpentylthio, Octylthio, Isooctylthio, 2-Ethylhexylthio, Nonylthio, Isononylthio, Decylthio, Isodecylthio, Undecylthio, Dodecylthio, Tridecylthio, Isotridecylthio, Tetradecylthio, Pentadecylthio, Hexadecylthio, Heptadecylthio, Octadecylthio, Nonadecylthio oder Eicosylthio.

Reste R¹, R⁵, Z³, Z⁴ und Z⁶ sind weiterhin, ebenso wie der Rest CR³R⁴R⁵ zusammen, z.B. Phenyl, 2-, 3- oder 4-Methylphenyl, 2-, 3-oder 4-Ethylphenyl, 2-, 3- oder 4-Propylphenyl, 2-, 3-oder 4-Isopropylphenyl, 2-, 3- oder 4-Butylphenyl, 2,4-Dimethylphenyl, 2-, 3- oder 4-Methoxyphenyl, 2-, 3- oder 4-Ethoxyphenyl, 2-, 3- oder 4-Isobutoxyphenyl, 2,4-Dimethoxyphenyl, 2-, 3- oder 4-Fluorphenyl, 2-, 3- oder 4-Chlorphenyl, 2-, 3- oder 4-Trifluormethylphenyl, 2-, 3- oder 4-Nitrophenyl oder 2-, 3- oder 4-Carboxylphenyl.

Reste R¹, R³, R⁴, Z³ und Z⁴ sind weiterhin z.B. 2-Carboxyethyl, 2-Methoxycarbonylethyl, Benzyl, 1- oder 2-Phenylethyl, 2-, 3-oder 4-Methylbenzyl, 2-, 3- oder 4-Methoxybenzyl, 2-, 3- oder 4-Chlorbenzyl, 2-, 3- oder 4-Nitrobenzyl, 3-Benzyloxypropyl, Phenoxymethyl, 6-Phenoxy-4-oxahexyl, 8-Phenoxy-4-oxaoctyl, 2-Hydroxyethyl, 2- oder 3-Hydroxypropyl, Fluormethyl, Chlormethyl, Difluormethyl, Dichlormethyl, Trifluormethyl, Trichlormethyl, 2-Fluorethyl, 2-Chlorethyl, 2,2,2-Trifluormethyl, Pentafluorethyl, Heptafluorpropyl, Nonafluorbutyl, 2-Cyanoethyl, 2- oder 3-Cyanopropyl, 2-Acetyloxyethyl, 2- oder 3-Acetyloxypropyl, 2-Isobutyryloxyethyl, 2- oder 3-Isobutyryloxypropyl, 2-Methoxycarbonylethyl, 2- oder 3-Methoxycarbonylpropyl, 2-Ethoxycarbonylethyl, 2- oder 3-Ethoxycarbonylpropyl, 2-Methylaminocarbonyloxyethyl, 2-Methoxycarbonyloxyethyl, 2- oder 3-Methoxycarbonyloxypropyl, 2-Ethoxycarbonyloxyethyl, 2- oder 3-Ethoxycarbonyloxypropyl, 2-Butoxycarbonyloxyethyl, 2- oder 3-Butoxycarbonyloxypropyl, 2-(2-Phenylethoxycarbonyloxy)ethyl, 2- oder 3-(2-Phenylethoxycarbonyloxy)propyl, 2-(2-Ethoxyethoxycarbonyloxy)ethyl oder 2-oder 3-(2-Ethoxyethoxycarbonyloxy)propyl.

Reste R⁵ sind weiterhin z.B. Methoxymethyl, Ethoxymethyl, Propoxymethyl, Butoxymethyl, Benzyl, 1- oder 2-Phenylethyl, Hydroxymethyl, 1- oder 2-Hydroxyethyl, 1-, 2- oder 3-Hydroxypropyl oder 4-Hydroxy-2-oxabutyl.

Reste R⁵ und CR³R⁴R⁵ sind weiterhin z.B. Thien-2-yl, Thien-3-yl, 3- oder 4-Methylthienyl oder 3- oder 4-Fluorthienyl.

Der Rest CR³R⁴R⁵ bedeutet weiterhin z.B. Fluormethyl, Chlormethyl, Difluormethyl, Dichlormethyl, Trifluormethyl, Trichlormethyl, 2-Fluorethyl, 2-Chlorethyl, 2,2,2-Trifluormethyl, Pentafluorethyl, Heptafluorpropyl, Nonafluorbutyl, Cyclopropyl, Cyclobutyl, Cyclopentyl, Methylcyclopentyl, Cyclohexyl, Methylcyclohexyl oder Cycloheptyl.

Reste Z³ und Z⁴ sind weiterhin z.B. Cyclopentyl, Methylcyclopentyl, Cyclohexyl, Methylcyclohexyl, Cycloheptyl, Allyl oder Methallyl.

Reste Z¹ sind z.B. Methylsulfonylamino, Ethylsulfonylamino, Propylsulfonylamino, Isopropylsulfonylamino, Butylsulfonylamino, Mono- oder Dimethylaminosulfonylamino, Mono- oder Diethylaminosulphonylamino, Mono- oder Dipropylaminosulfonylamino, Mono- oder Diisopropylaminosulfonylamino, Mono- oder Dibutylaminosulfonylamino oder (N-Methyl-N-ethylaminosulfonyl)amino.

Reste Z⁶ sind weiterhin, z.B. Fluor, Chlor, Brom, Benzyl, 2-Methylbenzyl, 2,4-Dimethylbenzyl, 2-Methoxybenzyl, 2,4-Dimethoxybenzyl, Methylamino, Ethylamino, Propylamino, Isopropylamino, Butylamino, Pentylamino, Hexylamino, Heptylamino, Octylamino, 2-Ethylhexylamino, Methylthio, Ethylthio, Propylthio, Isopropylthio oder Butylthio.

Reste R¹, R⁴, R⁵, Z¹, Z² und Z⁶ sind weiterhin z.B. Methoxy, Ethoxy, Propoxy, Isopropoxy, Butoxy, Isobutoxy, sec-Butoxy oder tert-Butoxy.

Reste R¹ sind weiterhin z.B. Pentyloxy, Isopentyloxy, Neopentyloxy, tert-Pentyloxy, Hexyloxy, Heptyloxy, 1-Ethylpentyloxy, Octyloxy, Isooctyloxy, 2-Ethylhexyloxy, Nonyloxy, Isononyloxy, Decyloxy, Isodecyloxy, Undecyloxy, Dodecyloxy, Tridecyloxy, Isotridecyloxy, tridecyloxy, Tetradecyloxy, Pentadecyloxy, Hexadecyloxy, Octadecyloxy, Nonadecyloxy oder Eicosyloxy.

Wenn Z³ und Z⁴ oder die unten genannten Reste L² und L³ zusammen mit dem sie verbindenden Stickstoffatom einen 5- oder 6-gliedrigen gesättigten heterocyclischen Rest, der gegebenenfalls weitere Heteroatome aufweist, bedeuten, so können dafür z.B. Pyrrolidinyl, Piperidinyl, Morpholinyl, Piperazinyl oder N-(C₁-C₄-Alkyl)piperazinyl in Betracht kommen.

Reste R⁶ sind z.B. Methoxycarbonyl, Ethoxycarbonyl, Propoxycarbonyl, Isopropoxycarbonyl, Butoxycarbonyl, Isobutoxycarbonyl oder sec-Butoxycarbonyl.

Bevorzugt sind Triazolopyridinfarbstoffe der Formel I, in der R⁶ für Cyano steht.

Weiterhin bevorzugt sind Triazolopyridinfarbstoffe der Formel I, in der R⁵ für C₁-C₅-Alkyl oder Phenyl steht.

Weiterhin bevorzugt sind Triazolopyridinfarbstoffe der Formel I, in der der Rest CR³R⁴R⁵ zusammen gegebenenfalls substituiertes Phenyl oder gegebenenfalls substituiertes Thienyl bedeutet.

Weiterhin bevorzugt sind Triazolopryridinfarbstoffe der Formel I, in der R¹ C₁-C₁₃-Alkyl, das gegebenenfalls durch C₁-C₆-Alkanoyloxy, C₁-C₈-Alkoxycarbonyl, dessen Alkylkette durch 1 oder 2 Sauerstoffatome in Etherfunktion unterbrochen sein kann, Phenyl oder C₁-C₄-Alkylphenyl substituiert ist und durch 1 oder 2 Sauerstoffatome in Etherfunktion unterbrochen sein kann, oder gegebenenfalls substituiertes Phenyl, bedeutet.

Insbesondere bevorzugt sind Triazolopyridinfarbstoffe der Formel I, in der R¹ Alkyl, Alkoxyalkyl, Alkanoyloxyalkyl oder Alkoxycarbonylalkyl, wobei diese Reste jeweils bis zu 12 Kohlenstoffatome aufweisen, gegebenenfalls durch Methyl substituiertes Benzyl oder gegebenenfalls durch Methyl substituiertes Phenyl bedeutet.

Weiterhin insbesondere bevorzugt sind Triazolopyridinfarbstoffe der Formel I, in der R² einen Rest der obengenannten Formel IIa, IIc, IIg oder IIi bedeutet, wobei
- Z¹: für Wasserstoff, C₁-C₄-Alkyl, C₁-C₄-Alkoxy oder C₁-C₈-Alkanoylamino,
- Z²: für Wasserstoff, Methyl, Methoxy oder Ethoxy,
- Z³ und Z⁴: unabhängig voneinander jeweils für Alkyl, Alkoxyalkyl, Alkanoyloxyalkyl oder Alkoxycarbonylalkyl, wobei diese Reste jeweils bis zu 12 Kohlenstoffatome aufweisen, Wasserstoff, gegebenenfalls durch Methyl substituiertes Benzyl oder Phenyl und
- Z⁶: für Wasserstoff, C₁-C₈-Alkyl, gegebenenfalls durch C₁-C₄-Alkyl oder C₁-C₄-Alkoxy substituiertes Phenyl, Benzyl oder Thienyl stehen.

Die erfindungsgemäßen Farbstoffe der Formel I können nach an sich bekannten Methoden hergestellt werden.

Beispielsweise können diejenigen Triazolopyridinfarbstoffe der Formel I, in der E CH bedeutet, durch Kondensation von Aldehyden der Formel III

R²-CHO (III),

in der R² die obengenannte Bedeutung besitzt mit Triazolopyridinen der Formel IVa oder IVb 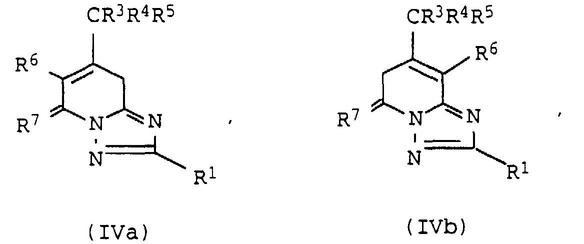 worin R¹, R³, R⁴, R⁵, R⁶ und R⁷ jeweils die obengenannte Bedeutung besitzen, erhalten werden.

Diejenigen Triazolopyridinfarbstoffe der Formel I, in der E Stickstoff bedeutet, können z.B. durch Kondensation von Nitrosoverbindungen der Formel V

R²-NO (V),

in der R² die obengenannte Bedeutung besitzt, oder durch oxidative Kupplung von Aminoverbindungen der Formel VI

R²-NH₂ (VI),

in der R² die obengenannte Bedeutung besitzt, mit den Triazolopyridinen IVa oder IVb erhalten werden.

Es ist jedoch auch möglich, die neuen Farbstoffe der Formel I auf dem aus der WO-A-95/21219 bekannten Weg herzustellen. So kann man beispielsweise eine Verbindung der Formel IVc oder IVd 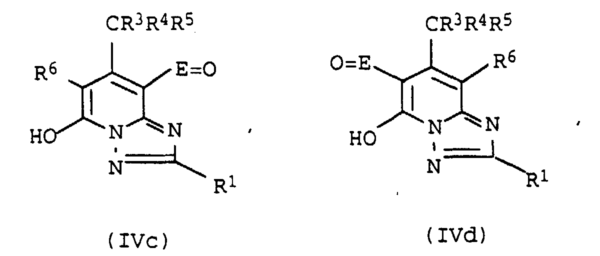 in denen E, R¹, R³, R⁴, R⁵ und R⁶ jeweils die obengenannte Bedeutung besitzen, mit einer Verbindung der Formel VII

R²-H (VII),

in der R² die obengenannte Bedeutung besitzt, kondensieren.

Ein weiterer Gegenstand der vorliegenden Erfindung sind neue Triazolopyridine der Formel IV 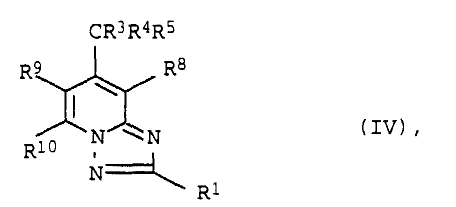 in der
- R¹: C₁-C₂₀-Alkyl, das gegebenenfalls substituiert ist und durch 1 bis 4 Sauerstoffatome in Etherfunktion unterbrochen sein kann, gegebenenfalls substituiertes Phenyl, Hydroxy, gegebenenfalls substituieres C₁-C₂₀-Alkoxy, Mercapto oder gegebenenfalls substituiertes C₁-C₂₀-Alkylthio,
- R³: Wasserstoff oder gegebenenfalls substituiertes C₁-C₄-Alkyl,
- R⁴: Wasserstoff oder gegebenenfalls substituiertes C₁-C₄-Alkyl oder C₁-C₄-Alkoxy,
- R⁵: C₁-C₈-Alkyl, das gegebenenfalls durch 1 oder 2 Sauerstoffatome in Etherfunktion unterbrochen ist und durch Phenyl oder Hydroxy substituiert sein kann, gegebenenfalls substituiertes Phenyl, gegebenenfalls substituiertes Thienyl oder C₁-C₄-Alkoxy, das gegebenenfalls durch ein Sauerstoffatom in Etherfunktion unterbrochen ist, oder der Rest CR³R⁴R⁵ zusammen C₃-C₇-Cycloalkyl, C₁-C₄-Halogenalkyl, gegebenenfalls substituiertes Phenyl oder gegebenenfalls substituiertes Thienyl,
- R⁸: Wasserstoff, Formyl, Nitroso, Cyano, C₁-C₄-Alkoxymethyl oder einen Rest der Formel CH₂-NL²L³, wobei L² und L³ unabhängig voneinander jeweils für Wasserstoff oder C₁-C₄-Alkyl, das gegebenenfalls durch C₁-C₄-Alkylimino unterbrochen ist, oder zusammen mit dem sie verbindenden Stickstoffatom für einen 5-oder 6-gliedrigen gesättigten heterocyclischen Rest stehen,
- R⁹: Wasserstoff, Cyano, Carbamoyl, Carboxyl, C₁-C₄-Alkoxycarbonyl oder Benzthiazolyl und
- R¹⁰: Hydroxy, Mercapto, Halogen, den Rest -NL²L³, wobei L² und L³ jeweils die obengenannte Bedeutung besitzen, oder den Rest einer CH-aciden Verbindung bedeuten.

Die exakte IUPAC-Bezeichnung des Grundkörpers, dem die neuen Triazolopyridine der Formel I zugrundeliegen, lautet 1,2,4-Triazolo[1,5-a]pyridin, wobei die folgende Bezifferung der Ringe gilt:

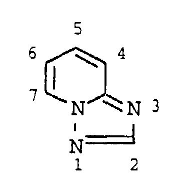

Die Verbindungen der Formel IV können in mehreren tautomeren Formen auftreten, die alle von den Patentansprüchen umfaßt werden. Beispielsweise können die Verbindungen mit R¹⁰ = Hydroxy u.a. in folgenden tautomeren Formen auftreten:

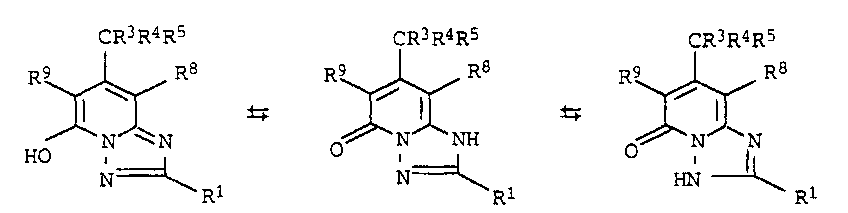

Wenn R¹⁰ in Formel IV einen Rest einer CH-aciden Verbindung bedeutet, so kann sich dieser Rest z.B. von Nitromethan, Nitroethan oder von CH-aciden Verbindungen der Formeln VIII bis XIII 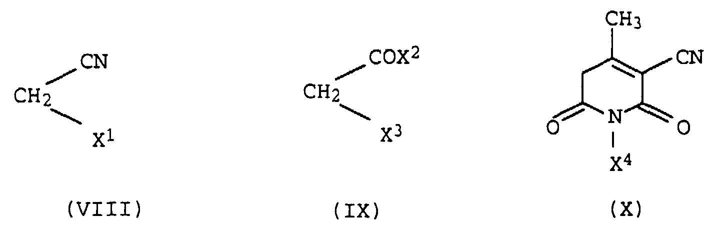 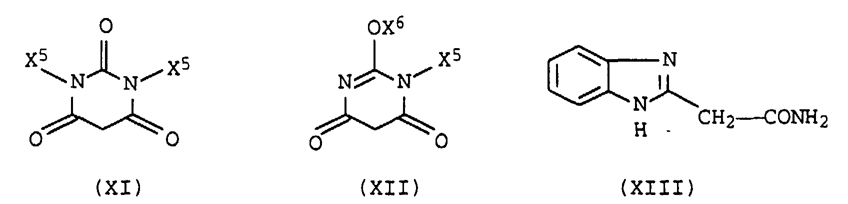 ableiten, wobei
- X¹: Cyano, Nitro, C₁-C₄-Alkanoyl, gegebenenfalls durch C₁-C₄-Alkyl, C₁-C₄-Alkoxy oder Halogen substituiertes Benzoyl, C₁-C₄-Alkylsulfonyl, gegebenenfalls durch C₁-C₄-Alkyl, C₁-C₄-Alkoxy oder Halogen substituiertes Phenylsulfonyl, Carboxyl, C₁-C₄-Alkoxycarbonyl, Phenoxycarbonyl, Carbamoyl, C₁-C₄-Mono- oder Dialkylcarbamoyl, gegebenenfalls durch C₁-C₄-Alkyl, C₁-C₄-Alkoxy oder Halogen substituiertes Phenylcarbamoyl, gegebenenfalls durch C₁-C₄-Alkyl, C₁-C₄-Alkoxy, Halogen oder Nitro substituiertes Phenyl, Benzthiazol-2-yl, Benzimidazol-2-yl, 5-Phenyl-1,3,4-thiadiazol-2-yl oder 2-Hydroxychinoxalin-3-yl,
- X²: C₁-C₄-Alkyl oder C₁-C₄-Alkoxy,
- X³: C₁-C₄-Alkoxycarbonyl, Phenylcarbamoyl oder Benzimidazol-2-yl,
- X⁴: Wasserstoff oder C₁-C₆-Alkyl,
- X⁵: Wasserstoff, C₁-C₄-Alkyl oder Phenyl und
- X⁶: C₁-C₄-Alkyl bedeuten.

Dabei sind insbesondere CH-acide Verbindungen der Formel VIII, IX oder XI zu nennen, wobei
- X¹: Cyano, Acetyl, Benzoyl, C₁-C₄-Alkoxycarbonyl, Phenoxycarbonyl, C₁-C₂-Monoalkylcarbonyl, Phenylcarbamoyl, Phenyl, Benzimidazol-2-yl, Benzthiazol-2-yl oder 5-Phenyl-l,3,4-thiazol-2-yl,
- X²: C₁-C₂-Alkoxy,
- X³: C₁-C₂-Alkoxycarbonyl oder Phenylcarbamoyl und
- X⁵: Methyl
bedeuten.

Reste R⁸ sind z.B. Aminomethyl, N-Mono- oder N,N-Dimethylaminomethyl, N-Mono- oder N,N-Diethylaminomethyl, N-Mono- oder N,N-Dipropylaminomethyl, Pyrrolidinomethyl, Piperidinomethyl, Morpholinomethyl, Piperazinomethyl, N-(C₁-C₄-Alkyl)piperazinomethyl, Methoxymethyl, Ethoxymethyl, Propoxymethyl, Isopropoxymethyl oder Butoxymethyl.

Weitere beispielhafte Aufzählungen von Resten, die in Formel IV auftreten, können den oben gemachten Ausführungen entnommen werden.

Bevorzugt sind Triazolopyridine der Formel IV, in der einer der beiden Reste R⁸ und R⁹ Wasserstoff und der andere Cyano bedeutet.

Weiterhin bevorzugt sind Triazolopyridine der Formel IV, in der R⁹ Cyano bedeutet.

Weiterhin bevorzugt sind Triazolopyridine der Formel IV, in der R⁵ C₁-C₅-Alkyl oder Phenyl bedeutet.

Weiterhin bevorzugt sind Triazolopyridine der Formel IV, in der der Rest CR³R⁴R⁵ zusammen gegebenenfalls substituiertes Phenyl oder gegebenenfalls substituiertes Thienyl bedeutet.

Weiterhin bevorzugt sind Triazolopyridine der Formel IV, in der R¹ C₁-C₁₃-Alkyl oder Phenyl bedeutet.

Besonders bevorzugt sind Triazolopyridine der Formel IV, in der R⁹ Cyano und R⁸ Wasserstoff bedeuten.

Die Herstellung der erfindungsgemäßen Triazolopyridine der Formel IV kann nach an sich bekannten Methoden geschehen, wie sie z.B. in der US-A-5 101 028 beschrieben sind.

Beispielsweise kann die Herstellung der Triazolopyridine der Formel IVe 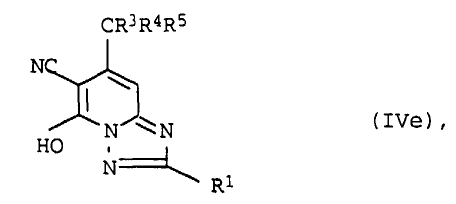 in der R¹, R³, R⁴ und R⁵ jeweils die obengenannte Bedeutung besitzen, durch Umsetzung eines Aminonitrils der Formel XIV 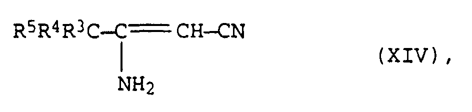 in der R³, R⁴ und R⁵ jeweils die obengenannte Bedeutung besitzen, mit einem Hydrazin der Formel XV

A-CO-NH-NH-CO-CH₂CN (XV),

in der A Amino oder den obengenannten Rest R¹ bedeutet, erfolgen.

Diejenigen Triazolopyridine der Formel IVf 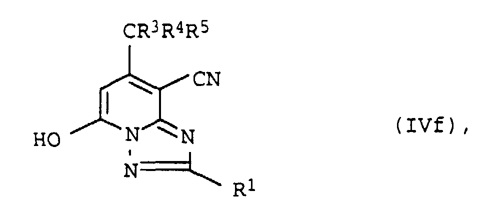 in der R¹, R³, R⁴ und R⁵ jeweils die obengenannte Bedeutung besitzen, kann z.B. durch Umsetzung eines Hydrazins der Formel XVI

A-CO-NH-NH-CO-CH₂COCR³R⁴R⁵ (XVI),

in der A, R³, R⁴ und R⁵ jeweils die obengenannte Bedeutung besitzen, mit Malodinitril erfolgen.

Die übrigen Triazolopyridine der Formel IV können aus den Verbindungen der Formel IVe oder IVf auf an sich bekanntem Wege hergestellt werden.

Beispielsweise kann der Substituent R⁸ mittels einer elektrophilen Substitutionsreaktion, z.B. Vilsmeier-Reaktion, oder Nitrosierung, gegebenenfalls mit anschließender Derivatisierung, eingeführt werden.

Die Cyanogruppe in Position 4 oder 6 läßt sich nach an sich bekannten Methoden in die Carbamoyl-, Carboxyl- oder C₁-C₄-Alkoxycarbonylgruppe überführen.

Durch Behandlung mit Säurehalogeniden, insbesondere Säurechloriden, z.B. Phosphoroxidtrichlorid, kann die Hydroxygruppe in Position 7 durch Halogen ersetzt werden.

Das Halogenatom wiederum läßt sich nach an sich bekannten Methoden durch CH-acide Verbindungen, Amine der Formel XVII 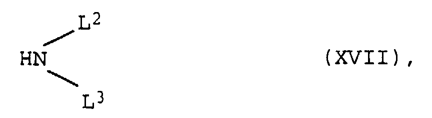 in der L² und L³ jeweils die obengenannte Bedeutung besitzen, oder Schwefelwasserstoff austauschen.

Die neuen Triazolopyridine der Formel IV sind wertvolle Zwischenprodukte, insbesondere für die Synthese der Farbstoffe der Formel I.

Ein weiterer Gegenstand der vorliegenden Erfindung ist ein Verfahren zur Übertragung von Farbstoffen von einem Träger auf ein mit Kunststoff beschichtetes Papier durch Diffusion oder Sublimation mit Hilfe einer Energiequelle, das dadurch gekennzeichnet ist, daß man einen Träger verwendet, auf dem sich ein oder mehrere Triazolopyridinfarbstoffe der Formel I befinden.

Zur Herstellung der für das erfindungsgemäße Verfahren benötigten Farbstoffträger werden die Farbstoffe der Formel I in einem geeigneten organischen Lösungsmittel oder in Mischungen von Lösungsmitteln mit einem oder mehreren Bindemitteln, gegebenenfalls unter Zugabe von Hilfsmitteln zu einer Druckfarbe verarbeitet. Diese enthält die Farbstoffe der Formel I vorzugsweise in molekular-dispers gelöster Form. Die Druckfarbe kann mittels einer Rakel auf den inerten Träger aufgetragen und die Färbung z.B. an der Luft oder mit einem Föhn getrocknet werden. Geeignete organische Lösungsmittel für die Farbstoffe der Formel I sind z.B. solche, in denen die Löslichkeit der Farbstoffe der Formel I bei einer Temperatur von 20°C größer als 1 Gew.-%, vorzugsweise größer als 5 Gew.-% ist.

Beispielhaft seien Ethanol, Propanol, Isobutanol, Tetrahydrofuran, Methylenchlorid, Methylethylketon, Cyclopentanon, Cyclohexanon, Toluol, Chlorbenzol oder deren Mischungen genannt.

Als Bindemittel kommen alle Resins oder Polymermaterialien in Betracht, die in organischen Lösungsmitteln löslich sind und die die Farbstoffe an den inerten Träger abriebfest zu binden vermögen. Dabei werden solche Bindemittel bevorzugt, die die Farbstoffmischung nach Trocknung der Druckfarbe an der Luft in Form eines klaren, transparenten Films aufnehmen, ohne das dabei eine sichtbare Auskristallisation der Farbstoffmischung auftritt.

Solche Bindemittel sind beispielsweise in der US-A-5 132 438 oder in den entsprechenden dort zitierten Patentanmeldungen genannt. Darüber hinaus sind gesättigte lineare Polyester zu nennen.

Bevorzugte Bindemittel sind Ethylcellulose, Ethylhydroxyethylcellulose, Polyvinylbutyral, Polyvinylacetat, Cellulosepropionat oder gesättigte lineare Polyester.

Das Gewichtsverhältnis Bindemittel : Farbstoff beträgt im allgemeinen 1 : 1 bis 10 : 1.

Als Hilfsmittel kommen z.B. Trennmittel in Betracht, wie sie in der US-A-5 132 438 oder den entsprechenden dort zitierten Patentanmeldungen genannt sind. Darüber hinaus sind besonders organische Additive zu nennen, welche das Auskristallisieren der Transferfarbstoffe bei Lagerung oder beim Erhitzen des Farbbandes verhindern, z.B. Cholesterin oder Vanillin.

Geeignete inerte Träger sind z.B. in der US-A-5 132 438 oder in . den entsprechenden dort zitierten Patentanmeldungen beschrieben. Die Dicke des Farbstoff-Trägers beträgt im allgemeinen 3 bis 30 µm.

Als Farbstoffnehmerschicht kommen prinzipiell alle temperaturstabilen Kunststoffschichten mit Affinität zu den zu transferierenden Farbstoffen in Betracht, z.B. modifizierte Polycarbonate oder Polyester. Weitere Einzelheiten dazu können z.B. aus der US-A-5 132 438 oder den entsprechenden dort zitierten Patentanmeldungen entnommen werden.

Die Übertragung erfolgt mittels einer Energiequelle, z.B. mittels eines Lasers oder eines Thermokopfes, wobei letzterer auf eine Temperatur von ≥ 300°C aufheizbar sein muß, damit der Farbstofftransfer im Zeitbereich t: 0 < t < 15 msec erfolgen kann. Dabei migriert der Farbstoff aus dem Transferblatt und diffundiert in die Oberflächenbeschichtung des Aufnahmemediums.

Die erfindungsgemäßen Farbstoffe der Formel I zeichnen sich beim Farbstofftransfer durch vorteilhafte anwendungstechnische Eigenschaften aus. Sie weisen eine hohe Löslichkeit im Farbband (gute Kompatibilität mit dem Bindemittel), eine hohe Stabilität in der Druckfarbe, eine gute Transferierbarkeit, eine hohe Bildstabilität (d.h. gute Lichtechtheit sowie gute Stabilität gegenüber Umwelteinflüssen, z.B. Feuchtigkeit, Temperatur oder Chemikalien) auf und erlauben eine flexible coloristische Anpassung an bereits vorgegebene subtraktive Grundfarben im Sinne einer optimalen Trichromie (höchstmögliche Brillanz von Grund- oder Mischfarben und tiefes neutrales Schwarz).

Die erfindungsgemäßen Farbstoffe der Formel I eignen sich weiterhin vorteilhaft zum Färben oder Bedrucken von synthetischen Materialien, z.B. von Polyestern, Polyamiden oder Polycarbonaten. Insbesondere zu nennen sind Materialien in textiler Form, wie Fasern, Garne, Zwirne, Maschenware, Webware oder Non-wovens aus Polyester oder Polyamid oder Polyester-Baumwolle-Mischgewebe. Sie eignen sich weiterhin zum Färben von Keratinfasern, z.B. Haare oder Pelze.

Die neuen Farbstoffe der Formel I eignen sich ebenfalls für die Herstellung von Farbfiltern, wie sie z.B. in der EP-A-399 473 beschrieben sind.

Schließlich können sie auch vorteilhaft als Farbmittel für die Herstellung von Tonern für die Elektrophotographie verwendet werden.

Die folgenden Beispiele sollen die Erfindung näher erläutern.

### A) Herstellung der Triazolopyridine

### Beispiel 1

In einem 1 l Kolben wurden zu einer Lösung von 50 g (1 mol) Hydrazinhydrat in 200 ml N,N-Dimethylacetamid bei einer Temperatur von 40 bis 50°C 124,45 g (1,1 mol) Cyanessigsäureethylester zugegeben und 1 h bei 40 bis 50°C gerührt. Anschließend erfolgte die Zugabe von 101 g (1 mol) Triethylamin. Man gab anschließend innerhalb von 4 h 161,9 g (1 mol) 2-Ethylhexansäure-chlorid bei 30°C hinzu und rührte 2 h bei dieser Temperatur nach. Anschließend gab man 1 g Titantetraethanolat und 123 g (1,5 mol) der Verbindung der Formel 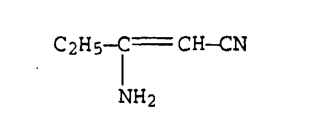 hinzu, erwärmte auf 120°C und destillierte dabei das gebildete Ethanol ab. Man rührte 1 h nach und erwärmte anschließend 6 h auf 160°C. Man gab dann die Reaktionsmischung in eine 70°C warme Lösung von 150 ml konz. Salzsäure in 2 l Wasser, rührte 1 h bei 70°C und saugte dann ab. Der Rückstand wurde mit 2 l Wasser gewaschen und unter vermindertem Druck getrocknet. Man erhielt 198 g der Verbindung der Formel

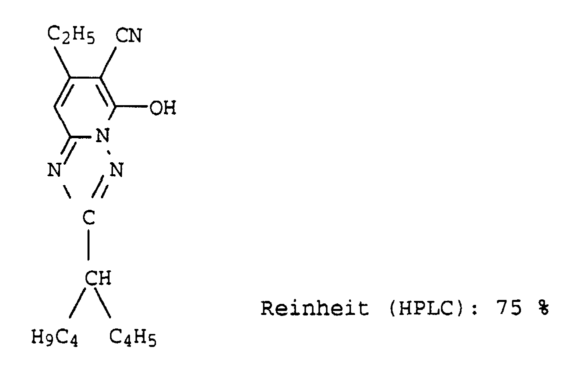

### Beispiel 2

In einem 1 l Kolben wurden zu einer Lösung von 50 g (1 mol) Hydrazinhydrat in 200 ml N,N-Dimethylacetamid bei einer Temperatur von 40 bis 50°C 124,45 g (1,1 mol) Cyanessigsäurethylester zugegeben und 1 h bei 40 bis 50°C gerührt. Anschließend erfolgte die Zugabe von 101 g (1 mol) Triethylamin. Man gab anschließend innerhalb von 4 h 161,9 g (1 mol) 2-Ethylhexansäurechlorid bei 30°C hinzu und rührte 2 h bei dieser Temperatur nach. Anschließend gab man 1 g Titantetraethanolat und 123 g (1,5 mol) der Verbindung der Formel 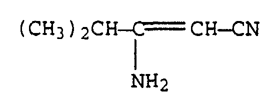 hinzu, erwärmte auf 120°C und destillierte dabei das gebildete Ethanol ab. Man rührte 1 h nach und erwärmte anschließend 6 h auf 160°C. Man gab dann die Reaktionsmischung in eine 70°C warme Lösung von 150 ml konz. Salzsäure in 2 1 Wasser, rührte 1 h bei 70°C und saugte dann ab. Der Rückstand wurde mit 2 1 Wasser gewaschen und unter vermindertem Druck getrocknet. Man erhielt 153 g der Verbindung der Formel

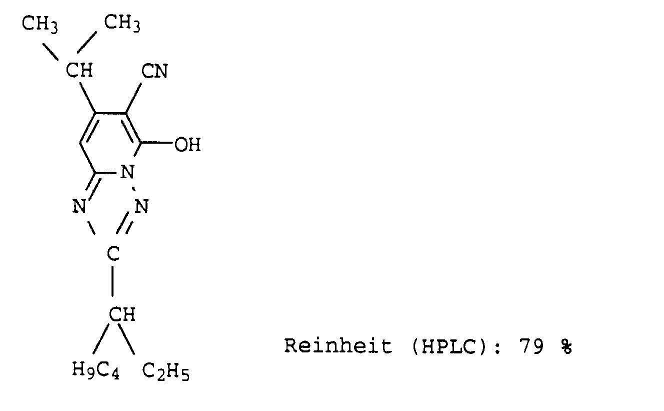

In analoger Weise können die in der folgenden Tabelle 1 aufgeführten Triazolopyridine hergestellt werden.

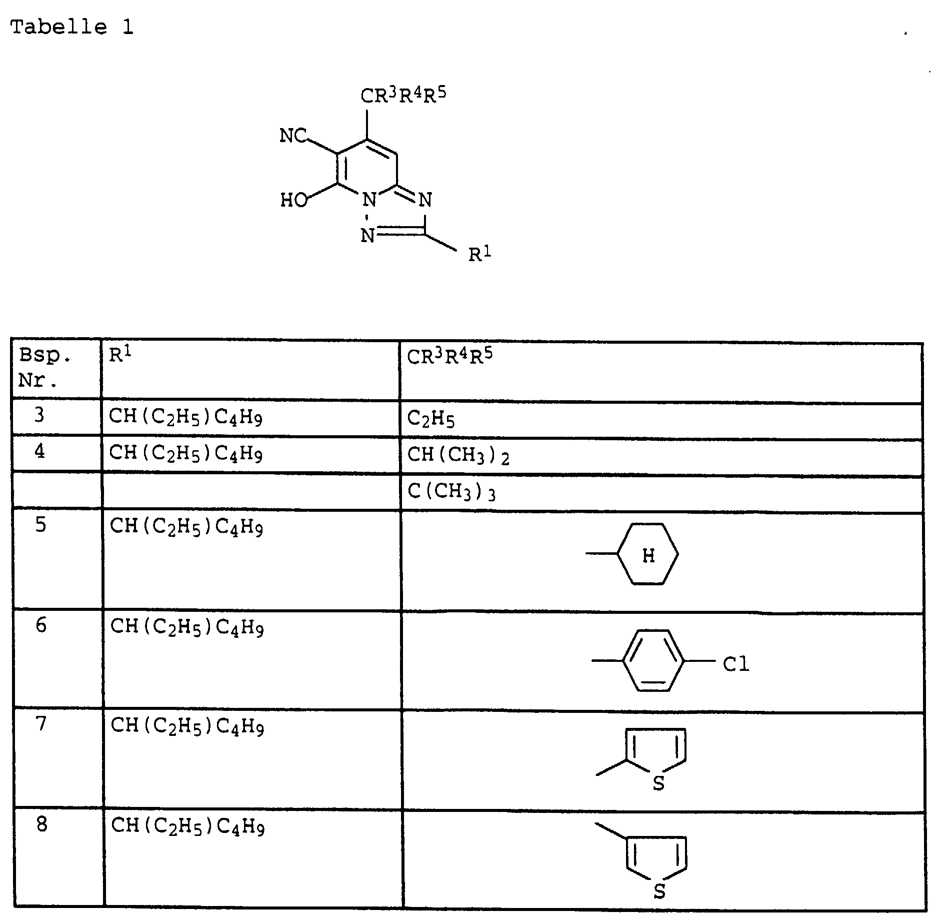

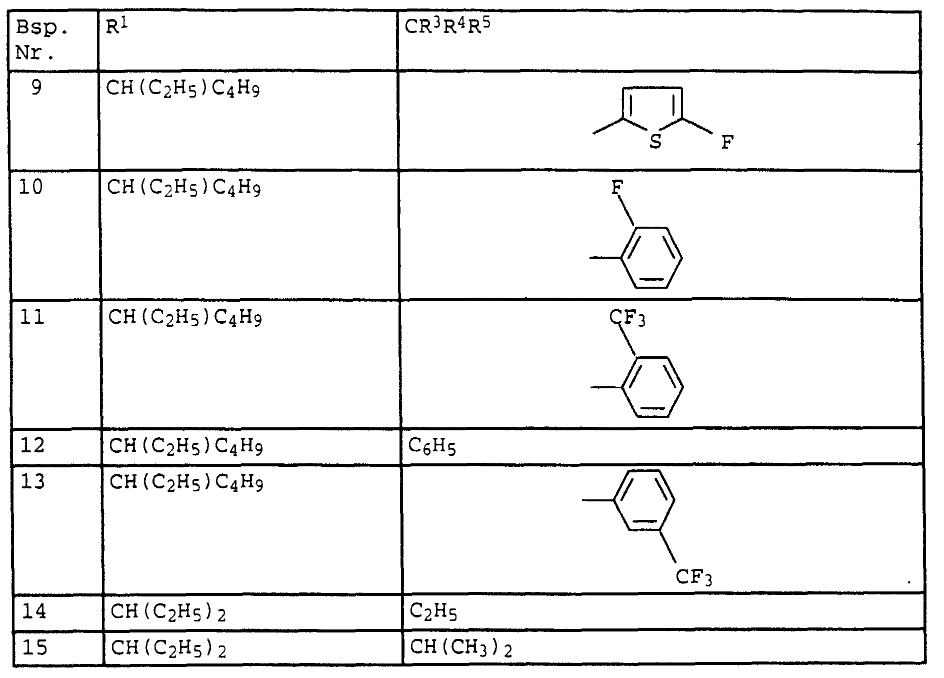

### B) Herstellung der Farbstoffe

### Beispiel 16

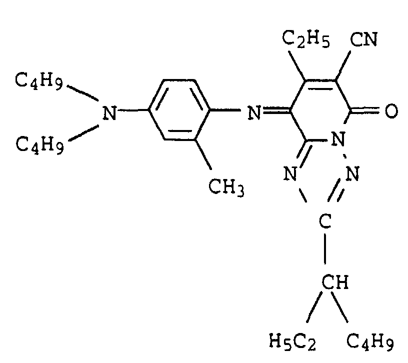

11 g N,N-Dibutyl-m-toluidin wurden wäßrig salzsauer mit Natriumnitrit bei 0°C bis 5°C nitrosiert. Nach 2 Stunden wurde mit 25 gew.-%igem Ammoniakwasser bei 0 bis 10°C alkalisch gestellt und mit 150 ml Methylenchlorid ausgeschüttelt. Die organische Phase wurde anschließend zu einer Suspension von 14 g der Verbindung der Formel 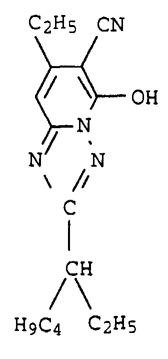 in Acetanhydrid bei Raumtemperatur unter Rühren zugegeben. Die Reaktion setzte sofort unter Wärmetönung ein. Es wurde noch 2 Stunden bei Raumtemperatur nachgerührt, auf 40°C erhitzt und Wasser zugegeben, um Acetanhydrid zu hydrolysieren. Die organische Phase wurde dreimal mit Wasser ausgeschüttelt und am Rotationsverdampfer eingeengt. Nach Aufrühren mit Wasser wurde mit wäßriger Natriumhydrogencarbonatlösung neutralisiert, abgesaugt, neutral gewaschen und bei Raumtemperatur getrocknet.
λₘₐₓ (Tetrahydrofuran = THF): 614 nm.

In analoger Weise wurden die folgenden Farbstoffe erhalten.

### Beispiel 17

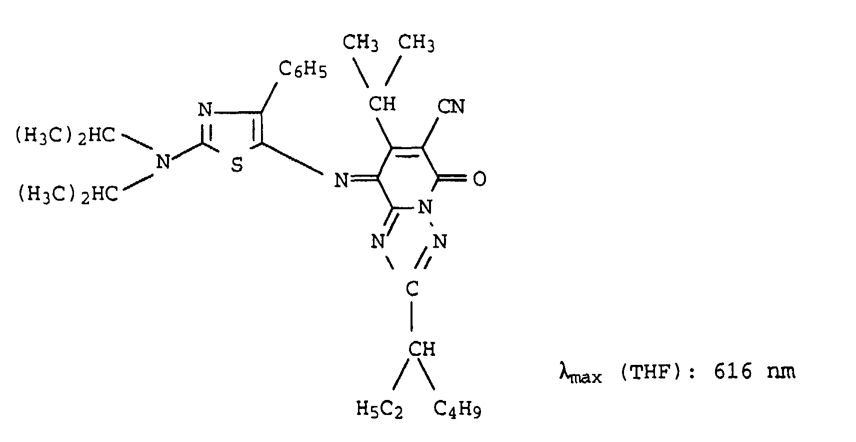

### Beispiel 18

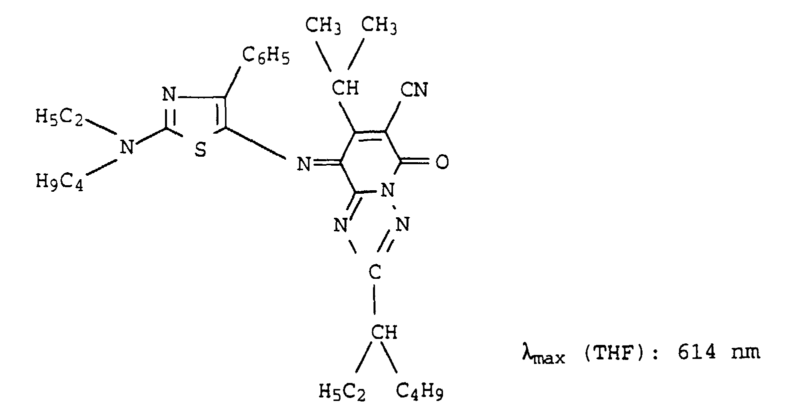

### Beispiel 19

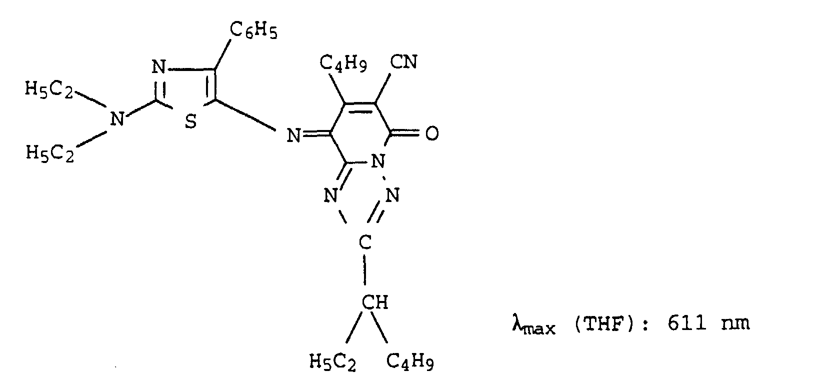

### Beispiel 20

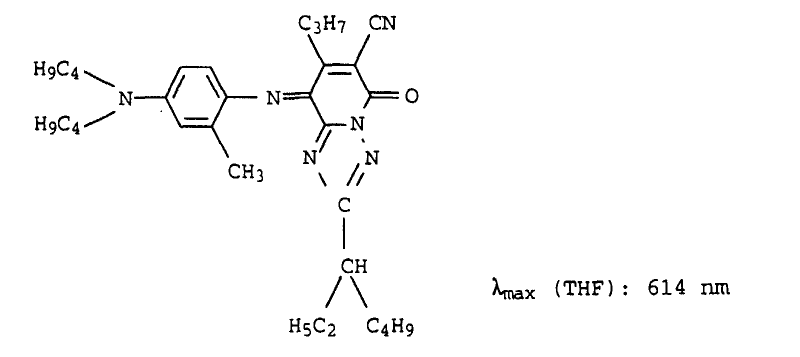

### Beispiel 21

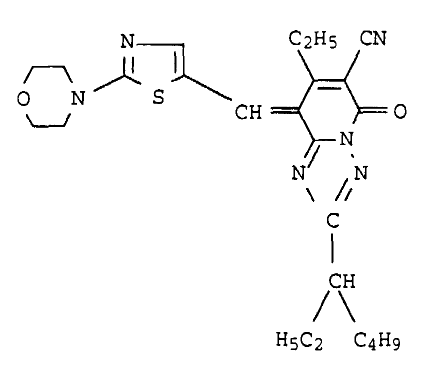

8,3 g 2-Morpholino-5-formylthiazol und 14,0 g der Verbindung der Formel 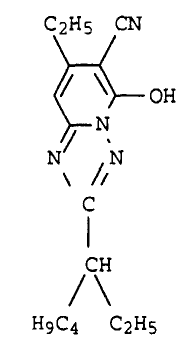 wurden in 100 ml Methylenchlorid gelöst. Nach Zugabe von 100 ml Acetanhydrid wurde Methylenchlorid abdestilliert und die Temperatur auf 120°C gesteigert. Nach Abkühlung unter Rühren auf 100°C fiel das Zielprodukt aus. Nach Zugabe von 100 ml Methanol wurde über Nacht gerührt, abgesaugt, mit Methanol gewaschen und getrocknet. λₘₐₓ (THF): 531 nm.

### Beispiel 22

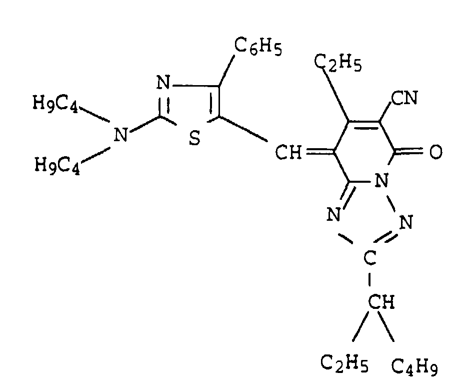

Zu einer Lösung von 88,4 g (0,31 mol) des Triazolopyridons der Formel 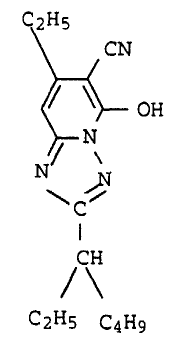 in 438 ml Eisessig und 438 ml konz. Salzsäure tropfte man bei 18 bis 20°C 79 g (0,31 mol) 27 gew.-%ige wäßrige Natriumnitritlösung innerhalb von 1 h zu und rührte 2 h bei Raumtemperatur nach. Anschließend gab man 325 ml Wasser bei 18 bis 20°C hinzu. Anschließend tropfte man bei 18 bis 20°C 72 g (0,25 mol) 2-Dibutylamino-4-phenylthiazol hinzu. Daraufhin wurden 438 ml 25 gew.-%ige Natronlauge bei einer Temperatur von 20 bis 30°C so zugegeben, daß ein pH-Wert von maximal 2 erreicht wurde. Die Suspension löste sich dabei auf. Man rührte 30 min bei 30°C nach, korrigierte dabei nochmals den pH-Wert, so daß der Wert von 2 gehalten wurde. Man erwärmte dann auf 60°C und rührte bei dieser Temperatur 2 h nach. Anschließend kühlte man auf 50°C ab. Die obere organische Phase wurde dann vor der wäßrigen Phase abgetrennt. Das Farbstofföl wurde bei 60°C in Methanol gelöst und durch Abkühlen auf 10°C zur Kristallisation gebracht. Nach einstündigem Rühren saugte man den ausgefallenen Farbstoff ab. Anschließend wusch man mit Methanol und dann mit Wasser nach. Der Farbstoff wurde anschließend bei 60°C getrocknet. Man erhielt 58,5 g (40%) Farbstoff unter vermindertem Druck (Reinheit 99 %).

### Beispiel 23

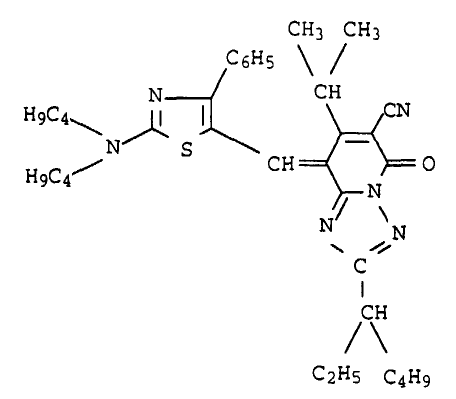

Zu einer Lösung von 88,4 g (0,31 mol) des Triazolopyridons der Formel 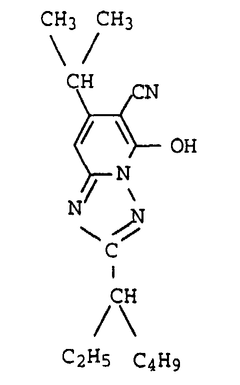 in 438 ml Eisessig und 438 ml konz. Salzsäure tropfte man bei 18 bis 20°C 79 g (0,31 mol) 27 gew.-%ige wäßrige Natriumnitritlösung innerhalb von 1 h zu und rührte 2 h bei Raumtemperatur nach. Anschließend gab man 325 ml Wasser bei 18 bis 20°C hinzu. Anschließend tropfte man bei 18 bis 20°C 72 g (0,25 mol) 2-Dibutylamino-4-phenylthiazol hinzu. Daraufhin wurden 438 ml 25 gew.-%ige Natronlauge bei einer Temperatur von 20 bis 30°C so zugegeben, daß ein pH-Wert von maximal 2 erreicht wurde. Die Suspension löste sich dabei auf. Man rührte 30 min bei 30°C nach, korrigierte dabei nochmals den pH-Wert, so daß der Wert von 2 gehalten wurde. Man erwärmte dann auf 60°C und rührte bei dieser Temperatur 2 h nach. Anschließend kühlte man auf 50°C ab. Die obere organische Phase wurde dann von der wäßrigen Phase abgetrennt. Das Farbstofföl wurde bei 60°C in Methanol gelöst und durch Abkühlen auf 10°C zur Kristallisation gebracht. Nach einstündigem Rühren saugte man den ausgefallenen Farbstoff ab. Anschließend wusch man mit Methanol und dann mit Wasser nach. Der Farbstoff wurde anschließend unter vermindertem Druck bei 60°C getrocknet. Man erhielt 48,0 g (30%) Farbstoff (Reinheit 99 %).

In ähnlicher Weise werden die folgenden Farbstoffe erhalten.

### Beispiel 24

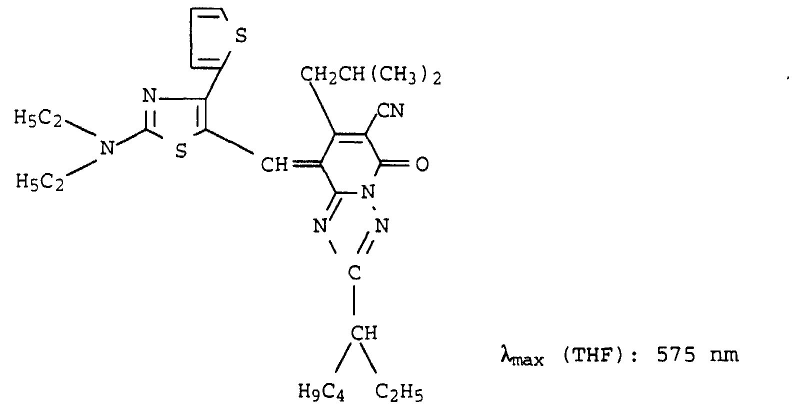

### Beispiel 25

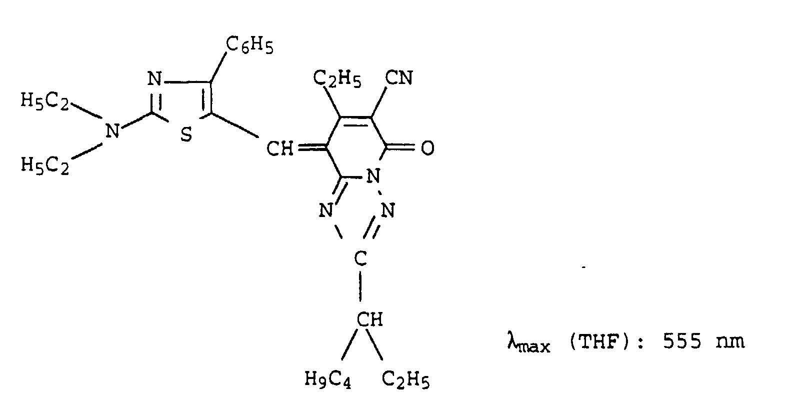

### Beispiel 26

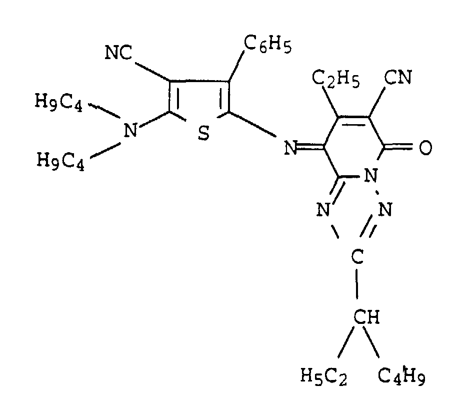

### Beispiel 27

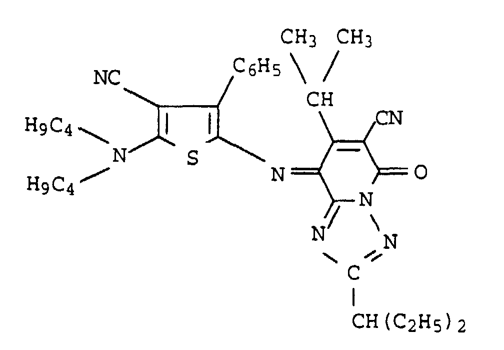

### Beispiel 28

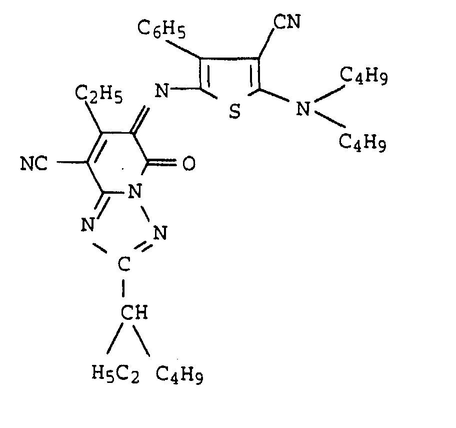

### C) Farbstofftransfer

### Allgemeine Vorschrift:

a) 10 g Farbstoff werden, gegebenenfalls unter kurzzeitigem Erwärmen auf 80 bis 90°C, in 100 g einer 10 gew.%igen Lösung eines Bindemittels auf Basis eines linearen Polyesters in ein Methylethylketon/Toluol/Cyclohexanon-Gemisch (4,5:2:2 v/v/v) eingerührt.
   Die Druckfarbe wird mit einer 6 µm Rakel auf eine Polyesterfolie von 6 µm Dicke, auf deren Rückseite eine geeignete Gleitschicht aufgebracht ist, aufgerakelt und mit einem Föhn 1 Minute trockengeblasen. Bevor das Farbband verdruckt werden kann, muß es mindestens 24 Stunden an der Luft nachtrocknen, da Restlösungsmittel den Druckvorgang beeinträchtigen können.
b) Die Farbbänder werden auf einer rechnergesteuerten Versuchsanordnung, die mit einem handelsüblichen Thermokopf ausgestattet ist, auf handelsüblichem Videoprintpapier (Typ VY-S der Firma Hitachi) verdruckt.
   Durch Veränderung der Spannung wird die Energieabgabe des Thermokopfs gesteuert, wobei die eingestellte Impulsdauer 7 ms beträgt und immer nur ein Impuls abgegeben wird. Die abgegebene Energie liegt zwischen 0,7 und 2,0 mJ/Dot.
   Da die Höhe der Anfärbung direkt proportional der zugeführten Energie ist, kann ein Farbkeil erzeugt und spektroskopisch ausgewertet werden.
   Aus der graphischen Auftragung der Farbtiefe gegen die zugeführte Energie je Heizelement wird der Q*-Wert (= Energie in mJ für den Extinktionswert 1) und die Steigung m in 1/mJ ermittelt.

Die erhaltenen Ergebnisse sind in der folgenden Tabelle 2 aufgeführt.

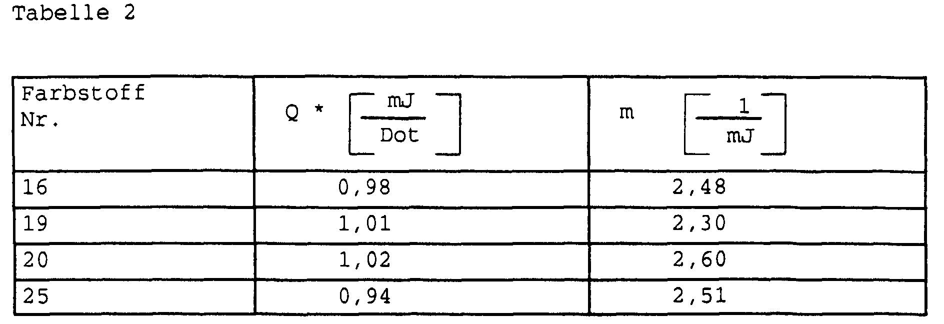

### D) Anwendung beim Färben

10 g Polyestergewebe werden bei einer Temperatur von 50°C in 200 ml einer Färbeflotte gegeben, die X Gew.-%, bezogen auf das Polyestergewebe, Farbstoff enthält und deren pH-Wert mittels Essigsäure auf 4,5 eingestellt ist. Man behandelt 5 min bei 50°C, steigert dann die Temperatur der Flotte innerhalb von 30 min auf 130°C, hält 60 min bei dieser Temperatur und läßt dann innerhalb von 20 min auf 60°C abkühlen.

Danach wird das ausgefärbte Polyestergewebe reduktiv gereinigt, indem man es 15 min in 200 ml einer Flotte, die 5 ml/l 32 gew.-%ige Natronlauge, 3 g/l Natriumdithionit und 1 g/l eines Anlagerungsproduktes von 48 mol Ethylenoxid an 1 mol Ricinusöl enthält, bei 65°C behandelt. Schließlich wird das Gewebe gespült, mit verdünnter Essigsäure neutralisiert, nochmals gespült und getrocknet.

Der Farbstoff Nr. 17 wurde in einer Menge (X) von 0,25 Gew.-% angewandt. Man erhielt hochbrillante grünstichig blaue Färbungen mit hervorragender Thermofixierechtheit.

## Patentansprüche

1. Triazolopyridinfarbstoffe der Formel I 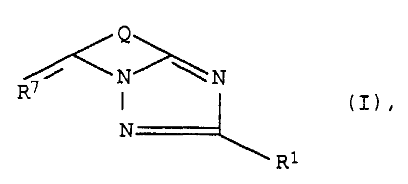 in der
R¹ C₁-C₂₀-Alkyl, das gegebenenfalls substituiert ist und durch 1 bis 4 Sauerstoffatome in Etherfunktion unterbrochen sein kann, gegebenenfalls substituiertes Phenyl, Hydroxy, gegebenenfalls substituiertes C₁-C₂₀-Alkoxy, Mercapto oder gegebenenfalls substituiertes C₁-C₂₀-Alkylthio,
Q einen Rest der Formel 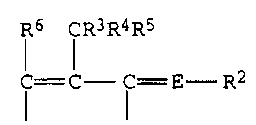 oder 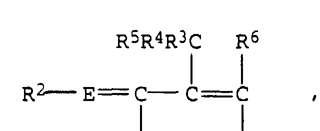 worin
R² für einen 5- oder 6-gliedrigen carbocyclischen oder heterocyclischen Rest, der benzoanelliert sein kann,
R³ für Wasserstoff oder gegebenenfalls substituiertes C₁-C₄-Alkyl,
R⁴ für Wasserstoff, gegebenenfalls substituiertes C₁-C₄-Alkyl oder C₁-C₄-Alkoxy,
R⁵ für C₁-C₈-Alkyl, das gegebenenfalls durch 1 oder 2 Sauerstoffatome in Etherfunktion unterbrochen ist und durch Phenyl oder Hydroxy substituiert sein kann, gegebenenfalls substituiertes Phenyl, gegebenenfalls substituiertes Thienyl oder C₁-C₄-Alkoxy, das gegebenenfalls nenfalls durch ein Sauerstoffatom in Etherfunktion unterbrochen ist, oder der Rest CR³R⁴R⁵ zusammen C₃-C₇-Cycloalkyl, C₁-C₄-Halogenalkyl, gegebenenfalls substituiertes Phenyl oder gegebenenfalls substituiertes Thienyl,
R⁶ für Cyano, Carbamoyl, Carboxyl, C₁-C₄-Alkoxycarbonyl oder Benzthiazolyl und
E für CH oder Stickstoff stehen, und
R⁷ Sauerstoff oder einen Rest der Formel 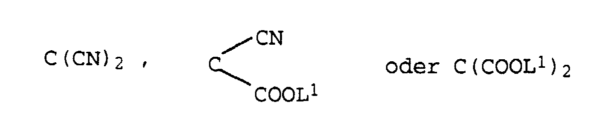
bedeuten, wobei L¹ jeweils für C₁-C₈-Alkyl, das gegebenenfalls durch 1 oder 2 Sauerstoffatome in Etherfunktion unterbrochen ist, steht.

2. Triazolopyridinfarbstoffe nach Anspruch 1, dadurch gekennzeichnet, daß R⁶ für Cyano steht.

3. Triazolopyridinfarbstoffe nach Anspruch 1, dadurch gekennzeichnet, daß R⁵ für C₁-C₅-Alkyl oder Phenyl steht.

4. Triazolopyridinfarbstoffe nach Anspruch 1, dadurch gekennzeichnet, daß der Rest CR³R⁴R⁵ zusammen gegebenenfalls substituiertes Phenyl oder gegebenenfalls substituiertes Thienyl bedeutet.

5. Triazolopyridinfarbstoffe nach Anspruch 1, dadurch gekennzeichnet, daß R¹ für C₁-C₁₃-Alkyl, das gegebenenfalls durch C₁-C₆-Alkanoyloxy, C₁-C₈-Alkoxycarbonyl, dessen Alkylkette durch 1 oder 2 Sauerstoffatome in Etherfunktion unterbrochen sein kann, Phenyl oder C₁-C₄-Alkylphenyl substituiert ist und durch 1 oder 2 Sauerstoffatome in Etherfunktion unterbrochen sein kann, oder gegebenenfalls substituiertes Phenyl steht.

6. Triazolopyridinfarbstoffe nach Anspruch 1, dadurch gekennzeichnet, daß R² sich von einer Komponente aus der Benzol-, Indol-, Chinolin-, Aminonaphthalin-, Pyrrol-, Aminothiazol-, Benzimidazol-, Benzthiazol-, Aminothiophen- oder Diaminopyridinreihe ableitet.

7. Verfahren zur Übertragung von Farbstoffen von einem Träger auf ein mit Kunststoff beschichtetes Papier durch Diffusion oder Sublimation mit Hilfe einer Energiequelle, dadurch gekennzeichnet, daß man einen Träger verwendet, auf dem sich ein oder mehrere Triazolopyridinfarbstoffe der Formel I gemäß Anspruch 1 befinden.

8. Verwendung der Triazolopyridinfarbstoffe gemäß Anspruch 1 zum Färben oder Bedrucken von synthetischen Materialien.

9. Triazolopyridine der Formel IV 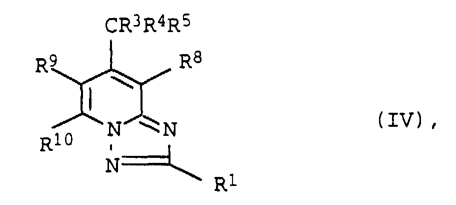 in der
R¹ C₁-C₂₀-Alkyl, das gegebenenfalls substituiert ist und durch 1 bis 4 Sauerstoffatome in Etherfunktion unterbrochen sein kann, gegebenenfalls substituiertes Phenyl, Hydroxy, gegebenenfalls substituiertes C₁-C₂₀-Alkoxy, Mercapto oder gegebenenfalls sübstituiertes C₁-C₂₀-Alkylthio,
R³ Wasserstoff oder gegebenenfalls substituiertes C₁-C₄-Alkyl,
R⁴ Wasserstoff oder gegebenenfalls substituiertes C₁-C₄-Alkyl oder C₁-C₄-Alkoxy,
R⁵ C₁-C₈-Alkyl, das gegebenenfalls durch 1 oder 2 Sauerstoffatome in Etherfunktion unterbrochen ist und durch Phenyl oder Hydroxy substituiert sein kann, gegebenenfalls substituiertes Phenyl, gegebenenfalls substituiertes Thienyl oder C₁-C₄-Alkoxy, das gegebenenfalls durch ein Sauerstoffatom in Etherfunktion unterbrochen ist, oder der Rest CR³R⁴R⁵ zusammen C₃-C₇-Cycloalkyl, C₁-C₄-Halogenalkyl, gegebenenfalls substituiertes Phenyl oder gegebenenfalls substituiertes Thienyl,
R⁸ Wasserstoff, Formyl, Nitroso, Cyano, C₁-C₄-Alkoxymethyl oder einen Rest der Formel CH₂―NL²L³, wobei L² und L³ unabhängig voneinander jeweils für Wasserstoff oder C₁-C₄-Alkyl, das gegebenenfalls durch C₁-C₄-Alkylimino unterbrochen ist, oder zusammen mit dem sie verbindenden Stickstoffatom für einen 5- oder 6-gliedrigen gesättigten heterocyclischen Rest stehen,
R⁹ Wasserstoff, Cyano, Carbamoyl, Carboxyl, C₁-C₄-Alkoxycarbonyl oder Benzthiazolyl und
R¹⁰ Hydroxy, Mercapto, Halogen, den Rest -NL²L³, wobei L² und L³ jeweils die obengenannte Bedeutung besitzen, oder den Rest einer CH-aciden Verbindung bedeuten.

10. Triazolopyridine nach Anspruch 9, dadurch gekennzeichnet, daß einer der beiden Reste R⁸ und R⁹ Wasserstoff und der andere Cyano bedeutet.

11. Triazolopyridine nach Anspruch 9, dadurch gekennzeichnet, daß R⁹ Cyano bedeutet.

12. Triazolopyridine nach Anspruch 9, dadurch gekennzeichnet, daß R⁹ Cyano und R⁸ Wasserstoff bedeuten.

13. Triazolopyridine nach Anspruch 9, dadurch gekennzeichnet, daß R⁵ C₁-C₅-Alkyl oder Phenyl bedeutet.

14. Triazolopyridine vom Anspruch 9, dadurch gekennzeichnet, daß der Rest CR³R⁴R⁵ zusammen gegebenenfalls substituiertes Phenyl oder gegebenenfalls substituiertes Thienyl bedeutet.

15. Triazolopyridine nach Anspruch 9, dadurch gekennzeichnet, daß R¹ C₁-C₁₃-Alkyl oder Phenyl bedeutet.

## Claims

1. A triazolopyridine dye of the formula I 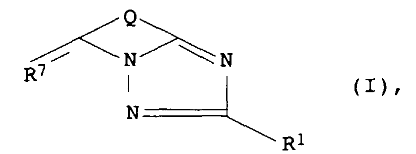 where
R¹ is C₁-C₂₀-alkyl which is unsubstituted or substituted and can be interrupted by 1 to 4 oxygen atoms in ether functionalities, unsubstituted or substituted phenyl, hydroxyl, unsubstituted or substituted C₁-C₂₀-alkoxy, mercapto or unsubstituted or substituted C₁-C₂₀-alkylthio,
Q is a radical of the formula 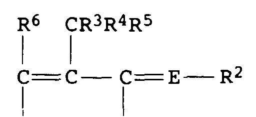 or 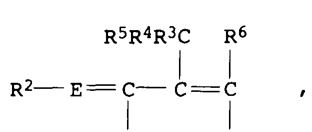 where
R² is a 5- or 6-membered carbocyclic or heterocyclic radical which can be benzo-fused,
R³ is hydrogen or unsubstituted or substituted C₁-C₄-alkyl,
R⁴ is hydrogen, unsubstituted or substituted C₁-C₄-alkyl or C₁-C₄-alkoxy,
R⁵ is C₁-C₈-alkyl which can be interrupted by 1 or 2 oxygen atoms in ether functionalities and can be phenyl- or hydroxyl-substituted, unsubstituted or substituted phenyl, unsubstituted or substituted thienyl or C₁-C₄-alkoxy which can be interrupted by an oxygen atom in ether functionalities, or the radical CR³R⁴R⁵ together is C₃-C₇-cycloalkyl, C₁-C₄-haloalkyl, unsubstituted or substituted phenyl or unsubstituted or substituted thienyl,
R⁶ is cyano, carbamoyl, carboxyl, C₁-C₄-alkoxycarbonyl or benzothiazolyl, and
E is CH or nitrogen, and
R⁷ is oxygen or a radical of the formula 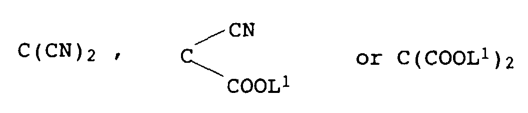
where L¹ is in each case C₁-C₈-alkyl which can be interrupted by 1 or 2 oxygen atoms in ether functionalities.

2. A triazolopyridine dye as claimed in claim 1, wherein R⁶ is cyano.

3. A triazolopyridine dye as claimed in claim 1, wherein R⁵ is C₁-C₅-alkyl or phenyl.

4. A triazolopyridine dye as claimed in claim 1, wherein the radical CR³R⁴R⁵ together is unsubstituted or substituted phenyl or unsubstituted or substituted thienyl.

5. A triazolopyridine dye as claimed in claim 1, wherein R¹ is C₁-C₁₃-alkyl which is unsubstituted or substituted by C₁-C₆-alkanoyloxy, C₁-C₈-alkoxycarbonyl whose alkyl chain can be interrupted by 1 or 2 oxygen atoms in ether functionalities, phenyl or C₁-C₄-alkylphenyl and which can be interrupted by 1 or 2 oxygen atoms in ether functionalities, or unsubstituted or substituted phenyl.

6. A triazolopyridine dye as claimed in claim 1, wherein R² is derived from a component of the benzene, indole, quinoline, aminonaphthalene, pyrrole, aminothiazole, benzimidazole, benzothiazole, aminothiophene or diaminopyridine series.

7. A process for transferring dyes from a support to a plastic-coated paper by diffusion or sublimation with the aid of an energy source, which comprises using a support on which there is or are one or more triazolopyridine dyes of the formula I as set forth in claim 1.

8. The use of the triazolopyridine dyes of claim 1 for dyeing or printing synthetic materials.

9. A triazolopyridine of the formula IV 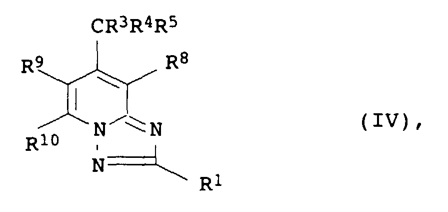 where
R¹ is C₁-C₂₀-alkyl which is unsubstituted or substituted and can be interrupted by 1 to 4 oxygen atoms in ether functionalities, unsubstituted or substituted phenyl, hydroxyl, unsubstituted or substituted C₁-C₂₀-alkoxy, mercapto or unsubstituted or substituted C₁-C₂₀-alkylthio,
R³ is hydrogen or unsubstituted or substituted C₁-C₄-alkyl,
R⁴ is hydrogen or unsubstituted or substituted C₁-C₄-alkyl or C₁-C₄-alkoxy,
R⁵ is C₁-C₈-alkyl which can be interrupted by 1 or 2 oxygen atoms in ether functionalities and can be phenyl- or hydroxyl-substituted, unsubstituted or substituted phenyl, unsubstituted or substituted thienyl or C₁-C₄-alkoxy which can be interrupted by an oxygen atom in ether functionalities, or the radical CR³R⁴R⁵ together is C₃-C₇-cycloalkyl, C₁-C₄-haloalkyl, unsubstituted or substituted phenyl or unsubstituted or substituted thienyl,
R⁸ is hydrogen, formyl, nitroso, cyano, C₁-C₄-alkoxymethyl or a radical of the formula CH₂-NL²L³, where L² and L³ are each independently of the other hydrogen or C₁-C₄-alkyl which can be interrupted by C₁-C₄-alkylimino, or together with the nitrogen atom connecting them are a 5- or 6-membered saturated heterocyclic radical,
R⁹ is hydrogen, cyano, carbamoyl, carboxyl, C₁-C₄-alkoxycarbonyl or benzothiazolyl, and
R¹⁰ is hydroxyl, mercapto, halogen, the radical -NL²L³, where L² and L³ are each as defined above, or the radical of an acidic-CH compound.

10. A triazolopyridine as claimed in claim 9, wherein one of the two radicals R⁸ and R⁹ is hydrogen and the other is cyano.

11. A triazolopyridine as claimed in claim 9, wherein R⁹ is cyano.

12. A triazolopyridine as claimed in claim 9, wherein R⁹ is cyano and R⁸ is hydrogen.

13. A triazolopyridine as claimed in claim 9, wherein R⁵ is C₁-C₅-alkyl or phenyl.

14. A triazolopyridine as claimed in claim 9, wherein the radical CR³R⁴R⁵ is together unsubstituted or substituted phenyl or unsubstituted or substituted thienyl.

15. A triazolopyridine as claimed in claim 9, wherein R¹ is C₁-C₁₃-alkyl or phenyl.

## Revendications

1. Colorants triazolopyridiniques de formule I 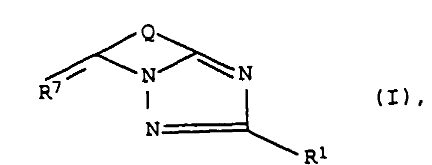 dans laquelle
R¹ représente un groupement alkyle en C₁-C₂₀, éventuellement substitué et pouvant être interrompu par 1 à 4 atomes d'oxygène en fonction éther, un groupement phényle éventuellement substitué, un groupement hydroxy, un groupement alcoxy en C₁-C₂₀ éventuellement substitué, un groupement mercapto ou alkylthio en C₁-C₂₀ éventuellement substitué,
Q représente un reste de formule 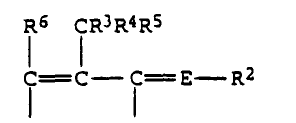 ou 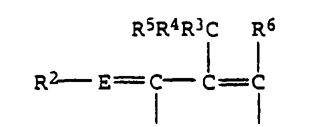 où
R² est mis pour un reste hétérocyclique ou carbocyclique à 5 ou 6 maillons, pouvant être benzocondensé,
R³ est mis pour un atome d'hydrogène ou un groupement alkyle en C₁-C₄ éventuellement substitué,
R⁴ est mis pour un atome d'hydrogène ou un groupement alcoxy en C₁-C₄ ou alkyle en C₁-C₄ éventuellement substitué,
R⁵ est mis pour un groupement alkyle en C₁-C₈, pouvant être interrompu par 1 ou 2 atomes d'oxygène en fonction éther et pouvant être substitué par des groupements phényle ou hydroxy, un groupement phényle éventuellement substitué, un groupement alcoxy en C₁-C₄ ou un groupement thiényle éventuellement substitué, pouvant être interrompu par un atome d'oxygène en fonction éther, ou bien le reste CR³R⁴R⁵ représente ensemble un groupement cycloalkyle en C₃-C₇, un groupement halogénoalkyle en C₁-C₄, un groupement phényle éventuellement substitué, ou un groupement thiényle éventuellement substitué,
R⁶ représente un groupement cyano, carbamoyle, carboxyle, (alcoxy en C₁-C₄)carbonyle, ou benzothiazolyle et
E représente CH ou un atome d'azote, et
R⁷ représente un atome d'oxygène ou un reste de formule 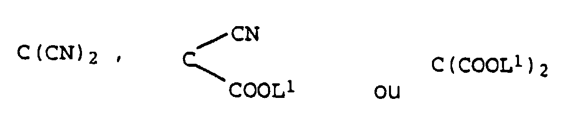
où L¹ représente à chaque fois un groupement alkyle en C₁-C₈, pouvant être interrompu par 1 ou 2 atomes d'oxygène en fonction éther.

2. Colorant triazolopyridinique selon la revendication 1, caractérisé en ce que R⁶ représente un groupement cyano.

3. Colorant triazolopyridinique selon la revendication 1, caractérisé en ce que R⁵ représente un groupement alkyle en C₁-C₅ ou un groupement phényle.

4. Colorant triazolopyridinique selon la revendication 1, caractérisé en ce que le reste CR³R⁴R⁵ représente ensemble un groupement phényle éventuellement substitué ou un groupement thiényle éventuellement substitué.

5. Colorant triazolopyridinique selon la revendication 1, caractérisé en ce que R¹ représente un groupement alkyle en C₁-C₁₃, pouvant être substitué par un groupement alcanoyloxy en C₁-C₆, (alcoxy en C₁-C₈)carbonyle, dont la chaîne alkyle peut être interrompu par 1 ou 2 atomes d'oxygène en fonction éther, phényle ou (alkyle en C₁-C₄)phényle et pouvant être interrompu par 1 ou 2 atomes d'oxygène en fonction éther, ou bien un groupement phényle éventuellement substitué.

6. Colorant triazolopyridinique selon la revendication 1, caractérisé en ce que R² dérive d'un composant provenant des séries du benzène, de l'indole, de la quinoléine, de l'aminonaphtalène, du pyrrole, de l'aminothiazole, du benzimidazole, du benzothiazole, de l'aminothiophène ou de la diaminopyridine.

7. Procédé de transfert de colorants d'un support sur un papier revêtu de matière plastique, par diffusion ou sublimation à l'aide d'une source d'énergie, caractérisé en ce que l'on utilise un support sur lequel se trouvent un ou plusieurs colorants triazolopyridiniques de formule I selon la revendication 1.

8. Utilisation de colorants triazolopyridiniques selon la revendication 1 pour la coloration ou l'impression de matériaux synthétiques.

9. Triazolopyridines de formule IV 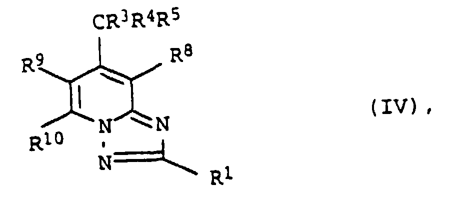 dans laquelle
R¹ représente un groupement alkyle en C₁-C₂₀, éventuellement substitué et pouvant être interrompu par 1 à 4 atomes d'oxygène en fonction éther, un groupement phényle éventuellement substitué, un groupement hydroxy, un groupement alcoxy en C₁-C₂₀ éventuellement substitué, un groupement mercapto ou (alkyle en C₁-C₂₀)thio éventuellement substitué,
R³ représente un atome d'hydrogène ou un groupement alkyle en C₁-C₄ éventuellement substitué,
R⁴ représente pour un atome d'hydrogène ou un groupement alcoxy en C₁-C₄ ou alkyle en C₁-C₄ éventuellement substitué,
R⁵ est mis pour un groupement alkyle en C₁-C₈, pouvant être interrompu par 1 ou 2 atomes d'oxygène en fonction éther et pouvant être substitué par des groupements phényle ou hydroxy, un groupement phényle éventuellement substitué, un groupement alcoxy en C₁-C₄ ou un groupement thiényle éventuellement substitué, pouvant être interrompu par un atome d'oxygène en fonction éther, ou bien le reste CR³R⁴R⁵ représente ensemble un groupement cycloalkyle en C₃-C₇, un groupement halogénoalkyle en C₁-C₄, un groupement phényle éventuellement substitué, ou un groupement thiényle éventuellement substitué,
R⁸ représente un atome d'hydrogène, un groupement formyle, nitroso, cyano, (alcoxy en C₁-C₄)méthyle ou un reste de formule CH₂-NL²L³, où L² et L³ représentent chacun indépendamment l'un de l'autre un atome d'hydrogène ou un groupement alkyle en C₁-C₄, pouvant être interrompu par un groupement (alkyle en C₁-C₄)imino, ou bien représentent ensemble avec l'atome d'azote qui les relie, un reste hétérocyclique saturé à 5 ou 6 maillons,
R⁹ représente un atome d'hydrogène, un groupement cyano, carbamoyle, carboxyle, (alcoxy en C₁-C₄)carbonyle, ou benzothiazolyle et
R¹⁰ représente un groupement hydroxy, mercapto, un atome d'halogène, le reste -NL²L³, où L² et L³ prennent chacun la signification susmentionnée, ou le reste d'un composé à CH-acide.

10. Triazolopyridines selon la revendication 9, caractérisées en ce que l'un des deux restes R⁸ et R⁹ représente un atome d'hydrogène et l'autre un groupement cyano.

11. Triazolopyridines selon la revendication 9, caractérisées en ce que R⁹ représente un groupement cyano.

12. Triazolopyridines selon la revendication 9, caractérisées en ce que R⁹ représente un groupement cyano et R⁸ un atome d'hydrogène.

13. Triazolopyridines selon la revendication 9, caractérisées en ce que R⁵ représente un groupement alkyle en C₁-C₅ ou un groupement phényle.

14. Triazolopyridines selon la revendication 9, caractérisées en ce que le reste CR³R⁴R⁵ représente ensemble un groupement phényle éventuellement substitué, ou un groupement thiényle éventuellement substitué.

15. Triazolopyridines selon la revendication 9, caractérisées en ce que R¹ représente un groupement alkyle en C₁-C₁₃ ou un groupement phényle.
